Europäisches Patentamt

European Patent Office

Office européen des brevets

(11) Publication number: **0 092 829**
**B1**

(12) **EUROPEAN PATENT SPECIFICATION**

(45) Date of publication of patent specification: **07.11.90**

(51) Int. Cl.⁵: **C 12 P 21/02, C 12 N 9/52, C 12 N 11/00**

(21) Application number: **83104036.5**

(22) Date of filing: **25.04.83**

(60) Divisional application 89123550.9 filed on 25/04/83.

(54) Process for semi-synthesis of human insulin and alkaline protease for use therein.

(30) Priority: 23.04.82 JP 68238/82
16.07.82 JP 123917/82
18.09.82 JP 162976/82
28.09.82 JP 169428/82

(43) Date of publication of application:
02.11.83 Bulletin 83/44

(45) Publication of the grant of the patent:
07.11.90 Bulletin 90/45

(84) Designated Contracting States:
AT BE CH DE FR GB IT LI LU NL SE

(56) References cited:
EP-A-0 017 938
EP-A-0 056 951
WO-A-82/04069
WO-A-83/00504
WO-A-83/01074
DE-A-1 915 970
GB-A-2 069 502
US-A-4 251 631

(73) Proprietor: WAKO PURE CHEMICAL INDUSTRIES, LTD.
10, Doshomachi-3-chome
Higashi-ku Osaka (JP)

(72) Inventor: Soejima, Masami
No. 847-1, Komatsu-cho
Tsuchiura-shi Ibaragi (JP)
Inventor: Masaki, Takeharu
No. 3978 Ami Ami-cho
Inashiki-gun Ibaragi (JP)
Inventor: Suzuki, Hideya
No. 2120, Mochida
Gyoda-shi Saitama (JP)
Inventor: Nagaoka, Jyoji
No. 37-3, Nishihara 2-chome
Shibuya-ku Tokyo (JP)
Inventor: Sakata, Yoshitsugu
No. 36-7, Hiyoshidai 2-chome
Otus-shi, Shiga-ken (JP)
Inventor: Shintani, Akinori
No. 43-4, Higashisonoda-cho 6-chome
Amagasaki-shi Hyogo (JP)
Inventor: Minamii, Nobuaki
No. 208, Hata
Neyagawa-shi Osaka (JP)

Courier Press, Leamington Spa, England.

## EP  0 092 829  B1

(56) References cited:

BIOCHEMICAL AND BIOPHYSICAL RESEARCH COMMUNICATIONS, vol. 92, no. 2, 29th January 1980, pages 396-402, Academic Press, Inc., New York, US; KAZUYUKI MORIHARA et al.: "Achromobacter protease I-catalyzed conversion of porcine insulin into human insulin"

CHEMICAL ABSTRACTS, vol. 95, no. 9, 31st August 1981, pages 346-347, no. 75965p, Columbus, Ohio, US; TAKEHARU MASAKI et al.: "Studies on a new proteolytic enzyme from Achromobacter lyticus M497-1. I. Purification and some enzymic properties" & BIOCHIM. BIOPHYS. ACTA 1981, 660(1), 44-50

CHEMICAL ABSTRACTS, vol. 92, no. 7, 18th February 1980, page 522, no. 56818q, Columbus, Ohio, US; & JP - A - 79 119 085 (TAKEDA CHEMICAL INDUSTRIES, LTD.) 14-09-1979

CHEMICAL ABSTRACTS, vol. 96, no. 11, May 1982, page 562, no. 179418z, Columbus, Ohio, US; RYONOSUKE MUNEYUKI et al.: "Enzyme immobilization and its application to the process of synthesizing human insulin from porcine insulin" & PEPT. CHEM. 1981 (Pub. 1982). 19th, 113-118

(72) Inventor: Matsuo, Tetsuya
No. 22, A4-704, Yamadanishi 1-chome
Suita-shi Osaka (JP)
Inventor: Sugiyama, Haruhiko
No. 20-5, Onosuimei 1-chome Shiga-cho
Shiga-gun Shiga (JP)
Inventor: Tokioka, Nobuyuki
Mogawa-Ryo Wako Pure Chemical Industries, Ltd.
No. 3-10, Takada-cho Amagasaki-shi Hyogo (JP)

(74) Representative: Hansen, Bernd, Dr.rer.nat. et al
Hoffmann, Eitle & Partner Patentanwälte
Arabellastrasse 4 Postfach 81 04 20
D-8000 München 81 (DE)

**Description**

The present invention relates to a process for semi-synthesis of human insulin. More particularly, the present invention relates to a process for semi-synthesis of human insulin from porcine insulin using as a catalyst Achromobacter protease Ia or water-soluble cross-linked Achromobacter protease Ia.

It is well known that insulin is a hormonal protein which is secreted from pancreas and plays an important role in reducing blood sugar and in sugar metabolism, and is essential for the treatment of diabetes. Animal-produced insulin is used; however such insulin is accompanided by side effects in human diabetes; and easy, economical and industrial production of human insulin has long been desired.

The different kinds as well as the sequence of amino acids which constitute insulin have already been established and it has long been known that the only difference between human insulin and porcine insulin is that the amino acid at the C-terminal of the B chain is threonine in human insulin and alanine in porcine insulin, and that the $B_{29}$ amino acid which is directly bound to the alanine or threonine is lysine. Some attempts to semi-synthesize human insulin from porcine insulin have been reported. It is also reported that *E. coli* producing human insulin was obtained utilizing genetic technology. It is recognized that these processes for preparation are epoch-making, and constitute advanced processes as compared to conventional chemical total synthesis or partial synthesis.

A process which comprises performing a step of cutting and removing alanine at $B_{30}$ of porcine insulin to obtain des-$B_{30}$-alanine insulin, performing as a second step a step of condensing protected threonine as the amino acid at $B_{30}$ and finally obtaining human insulin is disclosed in Japanese Patent Application (OPI) Nos. 135789/79 and 138391/80 (the term "OPI" as used herein refers to a "published unexamined Japanese patent application"), Japanese Patent Publication Nos. 18798/82 and 18799/82, etc. Further, a process for substituting protected threonine at $B_{30}$ in porcine insulin in the presence of trypsin is described in Japanese Patent Application (OPI) No. 135452/81, etc.

When examining these descriptions in detail, however, the concentration of insulins is high as compared to the amount of reaction solvent and fluidizability of the reaction liquid essential for the reaction is extremely poor; in particular, it is quite impossible to scale up the reaction of industrial production. Further, in these prior art processes in which very high amounts of organic solvents compared to the amount of water must be unavoidably used, dissolution of insulin is improved by the use of organic solvents, but such processes encounter a disadvantage in that the action of enzymes is greatly inhibited. In fact, when the same amount of organic solvents as that of water or higher amounts of organic solvents are mixed with water, the efficiency of enzyme is reduced to 1/10 to 1/100, and one is compelled to use expensive enzyme in a large excess. A further disadvantage is that an enzyme which is not used has to be discarded.

Further, *E. coli* producing human insulin is obtained by genetic technology and it is circulated as if such would be commercially available but a question is cast thereon by the informed people (for example, *Gendai Kagaku*, 1981, November, page 20, left column, published by Tokyo Kagaku Dojin, Tokyo). It is no exaggeration to say that this technology is not suitable for industrial preparation, i.e. because *E. coli* obtained by genetic technology does not produce insulin as an exo-enzyme but merely produces the same as an endo-enzyme so that the production yield is extremely small; further mutants are more fragile than mother strains and inferior in the competition for existence.

Synthesis of human insulin from porcine insulin utilizing an enzyme having a specific action to the carboxyl group of L-lysine is described in Japanese Patent Application (OPI) No. 119085/79 by Soejima and Masaki et al., and in *Biochemical and Biophysical Research Communication*, Vol. 92, page 362 (1980) by Morihara and Oka et al.

According to these conventional methods the enzyme is reacted with porcine insulin under the condition of pH 8.5, and desalanine insulin (DAI), composed of 29 amino acids in which the bond between lysine at the 29-position and alanine at the 30-position is decomposed and lysine is present on the carboxyl terminal, is obtained.

Next, when DAI is reacted with threonine-OBu$^t$ under the condition of pH 6.5 in the presence of the enzyme, a new peptide bond is synthesized between lysine and threonine and human insulin-OBu$^t$ is obtained.

By hydrolysis of human insulin-OBu$^t$, human insulin can be synthesized enzymatically and chemically.

EP—A—0 017 938 describes a process to produce human insulin from animal-insulin which consists in the condensation of a des-B30-insulin with a threonine derivative. The condensation is carried out with the use of A. lyticus protease I, while the deletion of the B30 amino acid is performed with either carboxypeptidase A or with A. lyticus protease I.

The not-prepublished document WO 83/01074 discloses processes for the semi-synthesis of human insulin involving the use of a protease of Achromobacter.

The methods applied are all liquid phase reactions using a water-soluble enzyme so that it is necessary to separate the enzyme from human insulin obtained; if the purification of insulin is insufficient, immunological side effects may occur as, for instance, the formation of anti-bodies; the latter represents a serious risk for the patient when insulin is administered over long periods of time.

It is a primary object of the present invention to provide a process for semi-synthesis of human insulin which is applicable on an industrial scale.

Further, it is an object of the present invention to provide a process for semi-synthesis of human insulin in which the protease used can be recovered easily and in a stable state. Thus, it is a further object of the present invention to provide a stabilized enzyme useful for the process of semi-synthesizing human insulin.

Also, it is an object of the present invention to provide a novel alkaline protease which possesses superior properties in the process of semi-synthesizing human insulin.

As a result of extensive investigations on a process applicable for industrial production, it has been found that human insulin containing at $B_{30}$ threonine having a protective group can be obtained from porcine insulin in one step, using an enzyme or the water-soluble cross-linked modification thereof which has a synthesis activity larger by two figures than trypsin, and specifically cuts only the acid peptide bond at the carboxyl group site of lysine, as compared to trypsin which cuts all of the acid peptide bonds at the carboxyl group site of the basic amino acids present in the polypeptide chains.

Another investigation has led to the discovery of a novel alkaline protease which can specifically hydrolyze the ester bond and the amide bond at the carboxyl group of L-lysine, and which is useful for the process of semi-synthesizing human insulin.

As a result of further investigation, it has been found that Achromobacter protease Ia can be stabilized without resort to immobilization and with retaining its solubility in water, and this water-soluble enzyme can be separated and purified with ease utilizing the large difference in the molecular weight.

The present invention is based on the above findings.

Therefore, the present invention provides a process for semi-synthesis of human insulin by preparing human insulin derivative containing carboxyl group-protected threonine at $B_{30}$ thereof from porcine insulin by using water as a reaction solvent characterized in that Achromobacter protease Ia or water-soluble cross-linked Achromobacter protease Ia is used as a catalyst.

In yet another embodiment, the present invention provides Achromobacter protease Ia as a novel alkaline protease capable of catalyzing the process for semi-synthesizing human insulin from porcine insulin.

Figure 1 represents the first electro-focusing experiment with Ampholine® (pH 3.5 to 10);

Figure 2 shows the second electro-focusing with Ampholine® (pH 4 to 6);

As a result of further detailed investigations as to a novel protease produced by *Achromobacter lyticus*, it has been found that a protease obviously different from Achromobacter protease L is obtained by isoelectric point focusing electrophoresis using Ampholite® having pH 3.5 to 10, and a novel protease has been isolated from the fraction and given the name *Achromobacter* protease Ia.

The present inventors have extensively investigated a method for obtaining the enzyme, its properties as well as a method for its utilization.

Thus, the present invention provides Achromobacter protease Ia having the characteristics as described below:

(i) Molecular weight: 30,000 (determined by a gel filtration method using Sephadex®G-75)

(ii) Isoeletric point: 5.3

(iii) pH reactivity: it has an optimum pH at pH 8.5 for the esterase activity and an optimum pH at pH 9.0 for the amidase activity, respectively.

(iv) Substrate reactivity: it selectively and specifically hydrolyzes the ester bond and the amide bond at the carboxyl group of L-lysine.

(v) Inhibitor: it is inhibited by diisopropyl phosphofluoride, tosyl-L-lysine chloromethyl ketone and phenylmethylsulfonyl chloride.

This protease is produced by the genus Achromobacter.

*Achromobacter lyticus* M 497-1 is deposited at the Foundation of Fermentation Research Institute, the American Type Culture Collection and the Agency of Industrial Science and Technology, Ministry of International Trade and Industry of Japan und IFO 12725, ATCC 21456 and FERM P-6718, respectively. The production of the enzyme is carried out by cultivating bacteria belonging to the genus Achromobacter in a culture medium. The culture medium may be a solid or liquid. Cultivation may be subjected to settled culture, but it is generally more advantageous that it is carried out under aerobic conditions using a liquid culture, such as shaking culture or aeration spinner culture. To a culture medium composition, any material may be added as long as the growth of the bacteria and the production of Achromobacter protease Ia are accelerated. That is, as carbon sources, examples include sugars such as glucose, saccharose, dextrin; as nitrogen sources, examples include organic or inorgnic nitrogen-containing compounds such as pepton, meat extract, yeast extract, dry yeast, soybean powders, casein, casamino acid, amino acids, ammonium salts. As inorganic salts, metals such as sodium, potassium, calcium, magnesium, may also be added in the form of phosphates, sulfates, carbonates, chlorides. Vitamins, nucleic acids and compounds related thereto may also be added for the purpose of accelerating the growth of the bacteria and the production of Achromobacter protease Ia.

Culture conditions such as liquid property of medium, culture temperature, amount of aeration, culture time, vary depending upon strains used, composition of medium, but, in short, are chosen and controlled appropriately such that an accumulated amount of the desired Achromobacter protease Ia becomes maximal. In most cases, the liquid property of a medium is about neutral, the culture temperature is between 20 and 35°C, preferably 25 and 30°C, aeration amounts from about 0.5 to 1.5 l/min per 1 liter of the medium, and the culture time is about 1 to 2 days.

4

Thus, a bacteria producing Achromobacter protease Ia is cultured and the same is secreted into and accumulated in the culture liquid.

To obtain the enzyme, conventional means for separation and purification can suitably be utilized and lead to a standard material having optional purity. That is, the product can be fractionally precipitated by salting out by the addition of salts, such as ammonium sulfate, to the supernatant or the filtrate obtained after removing the cells by a process such as centrifugal separation or filtration; or by adding a hydrophilic organic solvent, such as alcohols, acetone, to the supernatant or the filtrate. Further, the degree of purification can be enhanced by adsorption and desorption using, e.g., alumina, bentonite, calcium phosphate gel, activated charcoal, a chromatographic method using various ion exchange materials, a molecular sieve method using Sephadex®, biogel, singly or in suitable combination thereof. In addition, an isoelectric point precipitation method, a dialysis method, an electrophoresis method, a precipitation method using heavy metal ions, can also be employed. As above, Achromobacter protease Ia having optional purity can be separated. The method for obtaining the enzyme will be explained in more detail with reference to the examples described hereafter. Titers of amidase activity and esterase activity are determined by the following methods.

Method for measurement of amidase activity and unit thereof

The substrate solution comprises 1.3 ml of a 0.2 M 2-amino-2-methyl-1,3-propandiol buffer solution (pH 9.5) and 0.15 ml of an aqueous 2.5 mM benzoyl-DL-lysine-p-nitroanilide (hereafter referred to as Bz-Lys-p-NA) solution. After preliminarily warming to 30°C, 0.05 ml of an enzyme solution was added to the above solution and the mixture was reacted exactly 25 minutes. After completion of the reaction, 0.5 ml of a 45% (v/v) acetic acid aqueous solution was added to discontinue the reaction. Then, the reaction liquid was colorimetrically measured at 405 nm and its absorbancy was determined. As an enzyme unit, the amount of the enzyme that produced 1 μmole of p-nitroaniline per 1 minute at 30°C was defined as 1 unit (1 μ). The method for calculating the enzyme titer corresponds to the following equation.

$$\text{Activity (μ/ml)} = \Delta OD/\text{min} \times \frac{1}{9.62} \times \frac{2.0}{0.05} \times \text{dilution magnification}$$

Method for measurement of esterase activity and unit thereof

After preliminarily warming of 3.0 ml of a 40 mM tris-hydrochloride buffer solution (pH 8.0) containing 1 mM tosyl-L-lysine methyl ester (in the following referred to as TLME) at 30°C, 0.2 ml of an enzyme solution was added thereto and the change of absorbancy (ΔOD) was measured at 247 nm at 30°C. The amount of the enzyme that hydrolyzed 1 μmole of TLME per 1 minute at 30°C and pH 8.0 was defined as 1 unit. The method for calculating the enzyme titer corresponds to the following equation.

$$\text{Activity (μ/ml)} = \Delta OD/\text{min} \times \frac{1}{0.96} \times \frac{3.2}{0.2} \times \text{dilution magnification}$$

Enzymatical and chemical characteristics of Achromobacter protease Ia of the present invention will next be described.

(i) Molecular weight: 30,000 (as determined by gel filtration method using Sephadex®G-75).

(ii) Isoelectric point (by an isolectric fractionation method using Ampholine®;: pH 5.3

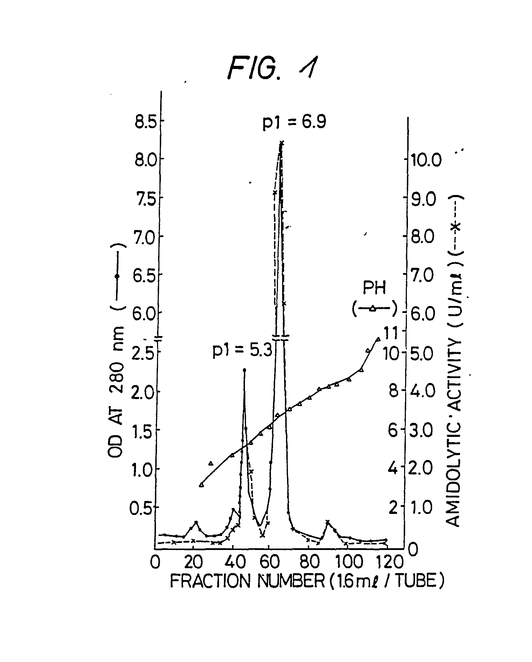

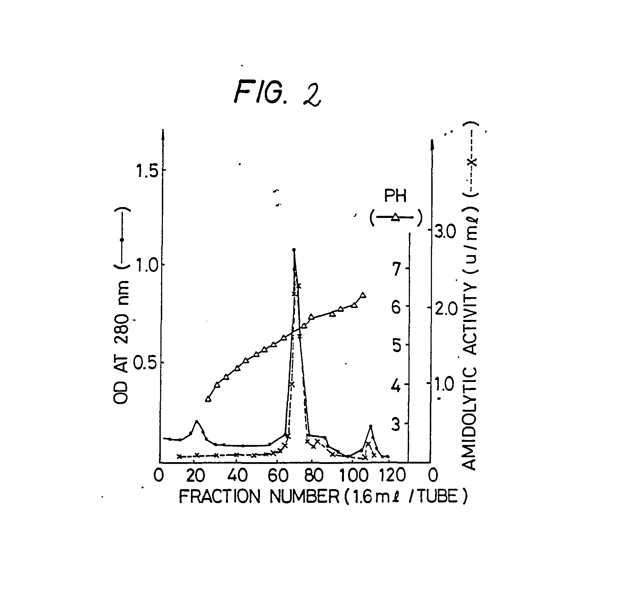

(iii) pH reactivity: it has an optimum pH at pH 9.0 for amidase activity (refer to Figure 1) with Bz-Lys-p-NA, and an optimum pH at pH 8.5 for esterase activity with TLME (refer to Figure 2).

(iv) pH stability: as shown in Figure 3, the enzyme is stable over a wide pH range from pH 5.0 to 11.0 at low temperature (treated at 4°C for 20 hours).

(v) Temperature stability: it is stable up to 40°C when heated at pH 9.0 for 15 minutes (refer to Figure 4).

(vi) Substrate reactivity: Amidase activity was measured with Bz-Lys-p-NA, N-benzoyl-L-arginine-p-nitroanilide (hereafter referred to as Bz-Arg-p-NA), L-lysine-p-nitroanilide (hereafter referred to as Lys-p-NA) and L-arginine-p-nitroanilide (hereafter referred to as Arg-p-NA) and esterase activity was measured with TLME and N-tosyl-L-arginine methyl ester (hereafter referred to as TAME), at pH 8 to 9.5 at 30°C to determine the Michaelis constant (Km) and molecular activity (Kcat) of the enzyme for each of the substrates, and the results shown in Table 1 were obtained. As is clear from this Table 1, this enzyme has an extremely high substrate specificity; the amide bond on the carboxyl group of L-lysine or L-arginine was affected in lysine but was not hydrolyzed in arginine. The enzyme hydrolyzed the ester bond of the carboxyl group in lysine or arginine; the action was strong for lysine but extremely slight for arginine.

EP 0 092 829 B1

TABLE 1

| Substrate | Quanti-tative analysis | Reaction pH | Km (mM) | Kcat (sec$^{-1}$) | Kcat/Km |
|---|---|---|---|---|---|
| Bz-Lys-p-NA | A | 9.5 | 0.101 | 1.73 | 17.13 |
| Bz-Arg-p-NA | A | 9.5 | not hydrolyzed | | |
| Lys-p-NA | A | 9.0 | 0.05 | 0.04 | 0.73 |
| Arg-p-NA | A | 9.0 | not hydrolyzed | | |
| TLME | B | 8.0 | 0.09 | 523.1 | 5812 |
| TAME | C | 8.0 | 0.75 | 0.28 | 0.374 |

\* Quantitative Analysis
A: Method by Tuppy et al. (*Z. Physiol. Cmem. 329*, 278 (1962))
B: Method by Schwert and Takenaka (*Biochem Biophys. Acta, 16*, 570 (1955))
C: Method by Makaki et al. (*Nippon Noka Shi, 51*, 195 (1977))

(vii) Influences of inhibitor and various metal salts: Influences of various inhibitors on amidase activity with Bz-Lys-p-NA are shown in Table 2. Further, influences of various metal ions on amidase activity with Bz-Lys-p-NA are shown in Table 3.

TABLE 2

| Inhibitor | Concentration (mM) | Relative activity (%) |
|---|---|---|
| No addition (control) | — | 100 |
| o-Phenanthroline | 1 | 99.3 |
| Ethylenediaminetetraacetate | 1 | 99.3 |
| L-cystein | 1 | 97.8 |
| Monoiodoacetate | 1 | 84.4 |
| Diisopropyl phosphofluoride | 1 | 70.1 |
| " | 10 | 53.2 |
| " | 20 | 27.5 |
| Phenylmethylsulfonyl fluoride | 2 | 18.3 |
| p-chloromercury benzoate | 0.1 | 94.8 |
| Tosyl-L-lysine chloromethyl ketone | 0.1 | 0 |
| Tosyl-L-arginine chloromethyl ketone | 0.1 | 79.1 |
| Dithiothreitol | 1 | 103.9 |
| Tosyl-L-phenylalanine chloromethyl ketone | 1 | 92.2 |

As shown in Table 2, the enzyme is a serine protease which undergoes inhibition by diisopropyl phosphofluoride, phenylmethylsulfonyl fluoride and tosyl-L-lysine chloromethyl ketone.

6

TABLE 3

| Metal salt | Concentration (mM) | Relative activity (%) |
|---|---|---|
| No addition (control) | — | 100 |
| $ZnCl_2$ | 1 | 44.1 |
| $CaCl_2$ | 1 | 102.2 |
| $NaCl_2$ | 1 | 99.0 |
| $MnCl_2$ | 1 | 101.3 |
| $NiCl_2$ | 1 | 83.8 |
| KCl | 1 | 90.6 |
| $MgCl_2$ | 1 | 95.3 |
| $HgCl_2$ | 0.1 | 109.4 |
| $CoCl_2$ | 1 | 98.1 |

As is clear from Table 3, this enzyme is inhibited by zinc ions.

As described above, this enzyme has a property of specifically hydrolyzing the amide bond and the ester bond of the carboxyl group of L-lysine so that it is applicable in to the fields of food chemistry, pharmaceuticals, clinical chemistry and biochemistry.

This enzyme can be used in solution as a monomer; a solution of this alternatively, this enzyme is cross-linked with cross-linking agents such as glutaraldehyde, diisocyanate, to convert the same into a water-soluble Achromobacter protease la polymer. It is needless to say that this enzyme is advantageously employed in the modification suitable for each utility or purpose. This novel enzyme has a characteristic substrate specificity that specifically acts on the peptide bond alone at the carboxyl group site of lysine and hydrolyzes the same; for this reason, this enzyme can be utilized concretely in the enzyme decomposition of peptides or proteins for the determination of configuration and order of amino acids, and the decomposition and synthesis of lysyl peptide.

The characteristic feature of the present invention lies in performing the desired amino acid exchange in one step by choosing water as a reaction solvent.

It is sufficient that the amount of water or an aqueous medium used as a reaction solvent be employed to an extent that the reaction mixture has fluidizability necessary for proceeding with the reaction. It is unnecessary to adhere to high concentrations incapable of stirring or fluidizing, as in the prior art. The process of the present invention results in a marked progress as to an easy procedure, smooth reaction and improvement in yield.

As organic solvents used, there is no limit as long as they are miscible with water, and methanol, ethanol, isopropanol, ethylene glycol, methyl cellosolve, acetone, dioxane, dimethylformamide, dimethyl sulfoxide, are exemplified as normal solvents. These organic solvents are employed such that the total volume of a mixture of one or more organic solvents be in the same amount as that of water or in an amount smaller than that of water, or no organic solvent is used, in which the process of the present invention lies.

The reaction is carried out at 50°C or lower but at 0°C or lower the reaction does not substantially proceed in a practical sense. The pH of the reaction liquid mixture is from 4 to 10, preferably about neutral. When the range of 4 to 10 in pH is not maintained, the reaction is slow and not practical.

For further improvement attempts have been made to stabilize Achromobacter protease without relying upon immobilization to give an enzyme which is soluble in water and is capable of being separated and purified with ease utilizing the large difference in the molecular weight.

In general, when enzyme is polymerized in any process, its active site changes and its enzyme activity is decreased; however, the polymer of the present invention showed no decrease in activity and extremely unexpected, useful result has been obtained.

The water-soluble cross-linked polymer of the present invention is a cross-linked polymer of Achromobacter protease la using a polyfunctional organic compound or the like, likewise a cross-linked polymer using polyamine as a spacer in combination, or a cross-linked polymer likewise using a water-soluble protein in combination; in any case, the resulting water-soluble cross-linked polymer of the present invention has a molecular weight of from about 400,000 to about 700,000, is soluble in water, extremely stable under environmental conditions over wide ranges and exhibits enzyme activity quite equivalent to that of the enzyme prior to polymerization under reaction conditions over wider ranges.

In cross-linking polymerization of the enzyme, the final concentration thereof in the reaction liquid is adjusted to generally 0.2 to 20 wt%, preferably 1 to 15 wt%. When the enzyme concentration becomes small, the yield of the cross-linked polymer of the present invention is decreased and practical nature is poor; when the concentration exceeds this limit, the product loses its water solubility. The thus-adjusted aqueous enzyme solution is used in combination with any one of the cross-linking agents of the present invention, spacers and water-soluble proteins as shown in Table 4 to allow to be reacted. Thus, the water-soluble cross-linked polymer of the present invention is obtained.

TABLE 4

| Achromobacter protease Ia | Cross-linking agent | Spacer | Water-soluble protein |
|---|---|---|---|
| o | o | — | — |
| o | o | o | — |
| o | o | — | o |
| o | o | Q | o |

As the cross-linking agents of the present invention, polyaldehydes such as glutaraldehyde; polyisocyanates such as hexamethylenedithisisocyanate, hexamethylenediisocyanate, toluenediisocyanate; are generally employed, and it is preferred that the cross-linking agent be used in a concentration of about 1 to 5%. When the concentration is too high, the reaction becomes vigorous and insolubilization or the like occurs; when the concentration is too low, the yield is decreased. Examples of the spacers of the present invention incldue polyamines such as spermine, spermidine, and, as water-soluble proteins of the present invention, proteins such as albumin, water-soluble gelatin and the like are preferred since they are supplied particularly inexpensively on an industrial scale. Any of the combinations as described in Table 4 is performed and these mixtures are incubated generally around neutral range of about 30°C for several minutes to 30 hours to cause cross-linking polymerization. Thereafter, raw materials are removed by dialysis, gel filtration is performed, ultra-filtration is performed, ultracentrifugation is performed, electrophoresis using polyacrylamide gel is utilized, or, isoelectric point electrophoresis such as ampholine, is utilized; in any case, the product can easily be isolated. The thus obtained Achromobacter protease Ia cross-linked polymer of the present invention can be used for enzyme reaction as it is, can be stored in a buffer solution having a suitable pH at 30°C or lower, or, can be stored as a freeze dried product; in any case, the polymer exhibits extremely effective enzyme reaction activity appropriately depending upon necessity.

The water-soluble cross-linked polymer of Achromobacter protease Ia in accordance with the present invention shows extremely high activity and specificity; further, pH stability, heat stability and stability in various media are markedly high as compared to the enzyme prior to polymerization.

The water-soluble cross-linked polymer of Achromobacter protease Ia in accordance with the present invention strongly acts on the bond of the carboxyl group of lysine but hardly acts on arginine and does not hydrolyze the bond of other amino acids.

In the case of using the cross-linked polymer in accordance with the present invention, the polymer mixed with its substrate; after reacting the mixture under optimum conditions, the cross-linked polymer of the present invention is recovered by gel filtration or ion exchange materials, etc. Such an easy separation and recovery is one of advantages of the present invention and, at the same time, the cross-linked polymer of the present invention can be repeatedly used because no loss is found in its enzyme activity.

Due to such a specific activity and stability, it is a matter of course that the polymer can be utilized for enzyme decomposition of peptides in termination of amino acid configuration, decomposition and synthesis and lysyl peptides, etc., and the present inventors have established a process for semi-synthesizing human insulin using the cross-linked polymer of the present invention.

As methods for converting porcine insulin into human insulin, there are chemical methods as described in *Science*, Vol. 177 (1972), page 623, authored by Luttenberg, *Physiological Chemistry*, Vol. 357 (1976), page 759, authored by Geiger, et al., etc., and enzyme methods as described in Japanese Patent Application (OPI) Nos. 135789/79 and 18799/80, etc. The former is complicated, produces many by-products and provides low yields, and the latter is unavoidably expensive from an industrial viewpoint while some improvement was made since difficulties are seen in the amount of enzyme used and recovery of the enzyme or in the use of organic solvents.

The process of the present invention is an epoch-making process for semi-synthesizing human insulin which solves all of these drawbacks. Due to water solubility of the cross-linked polymer of the enzyme in accordance with the present invention, the reaction can be performed in a homogeneous system; and due to extremely high stability, it is possible to recover and repeatedly use the polymer without losing the activity of the enzyme action; or, the enzyme reaction can be carried out in high yield both in water alone or in the system in which organic solvents miscible with water are used in combination over wide ranges. Further, due to high molecular weight of the cross-linked polymer of the present invention, separation and purification of the product as well as recovery of the cross-linked polymer of the present invention are extremely advantageous since the product can easily be taken out by, for example, a molecular sieve gel filtration method.

To utilize the cross-linked polymer of the present invention for semi-synthesis of human insulin, there are two reactions. One comprises reacting porcine insulin, L-threonine in which the carboxyl group is protected or unprotected (hereafter represented by Thr-OR, wherein R is hydrogen or substituted or unsubstituted alkyl or aralkyl), and the cross-linked polymer in accordance with the present invention to prepare a human insulin derivative having Thr-OR at $B_{30}$ and then converting the derivative into human insulin in a conventional manner; the other comprises incubating porcine insulin and the cross-linked polymer of the present invention, cutting $B_{30}$ from the resulting desalanine insulin (hereafter referred to as DAI), then incubating DAI, Thr-OR and the cross-linked polymer of the present invention to prepare human insulin derivatve having Thr-OR at $B_{30}$ (hereafter referred to as $B_{30}$ RO-Thr-I) and then converting the derivative into human insulin in a conventional manner.

In any case, water alone or one or more organic solvents miscible with water for increasing the solubility of raw materials in combination can be used as reaction solvents. With respect to the amount of the organic solvents used in combination, there is no particular limit. Examples of organic solvents used include methanol, ethanol, isopropanol, ethylene glycol, methyl cellosolve, acetone, dioxane, dimethylformamide, dimethyl sulfoxide. The stability of the water-soluble cross-linked polymer in accordance with the present invention in these solvents is shown in Table 5 in comparison with the enzyme prior to polymerization, wherein there is no fear that the activity is reduced.

TABLE 5

Stability in solvent (stored at 30°C in solvent and indicated by a residual activity rate)

| Solvent (examined as a 50% aqueous soln.) | Time | | | | | | | |
|---|---|---|---|---|---|---|---|---|
| | After 1 hour | | After 24 hours | | After 48 hours | | After 192 hours | |
| | A (%) | B (%) | A (%) | B (%) | A (%) | B (%) | A (%) | B (%) |
| Methanol | 105 | 110 | 103 | 113 | 103 | 113 | 105 | 103 |
| Ethanol | 110 | 100 | 107 | 114 | 105 | 112 | 106 | 101 |
| Isopropanol | 104 | 99 | 107 | 114 | 99 | 110 | 103 | 101 |
| Ethylene glycol | 105 | 100 | 110 | 113 | 109 | 115 | 106 | 101 |
| Acetone | 116 | 114 | 120 | 120 | 112 | 122 | 130 | 126 |
| Dimethylformamide | 99 | 104 | 100 | 110 | 99 | 115 | 105 | 97 |
| Dimethyl sulfoxide | 97 | 98 | 92 | 93 | 94 | 97 | 96 | 92 |
| Water | 100 | 112 | 98 | 110 | 92 | 106 | 95 | 88 |

A: Cross-linked polymer of the present invention (obtained in Example 1)
B: Enzyme prior to polymerization

The protected threonine is represented by Thr-OR. That is, the protective group is to protect the carboxyl group of threonine. It is unavoidable to protect other functional groups at the same time but in the case of using threonine having, e.g., the hydroxyl group being simultaneously protected, there is a possibility accompanying a disadvantage that requires an additional step for obtaining the desired human insulin. R in Thr-OR is substituted or unsubstituted alkyl or aralkyl and examples thereof include methyl, ethyl, propyl, isopropyl, tertiary butyl, chloroethyl, pentyl, octyl, 2-(propylsulfonyl)ethyl, benzyl, phenylethyl, p-methylphenethyl, 2,4,6-trimethylbenzyl.

The reaction temperature is below 50°C but preferably from 20 to 40°C. The pH of the reaction system is from 4 to 10, particularly preferably 5 to 8. It is desired that the molar concentration ratio of Thr-OR to porcine insulin or DAI be large but generally sufficient to be about 1:5 to 1:1000. As buffering agents of the reaction liquid, trishydroxymethylaminomethane, citrate, phosphate buffer solutions, etc., are employed. It is preferred that the concentration of the cross-linked polymer in accordance with the present invention in the reaction liquid be about from 0.1 to 10 mg/ml. The reaction time is generally from 3 to 72 hours, in most cases, 6 to 24 hours.

The obtained $B_{30}RO$-Thr-I is converted into human insulin in a conventional manner ordinarily used for peptide synthesis, for example, when R is t-butyl, i.e., $B_{30}Bu^tO$-Thr-I is used, by removing t-butyl through treatment with anisole-trifluoroacetic acid. Also when R in Thr-OR is hydrogen, i.e., threonine is used, it was confirmed by an experiment using labelled threonine that the reaction of peptide exchange proceeded, but it is not easy to separate and purify the product from raw porcine insulin and such is not recommendable.

Human insulin semi-synthesized by the process of the present invention can be converted into medical preparations in a conventional manner, which are administered to patients, for example, as drugs for treating diabetes. The process of the present invention which provides human insulin extremely efficiently is industrially utilizable without any disturbance and greatly contributes to the art. Hereafter, some examples and reference examples are shown but these examples are not deemed to limit the present invention. In examples showing cross-linking polymerization, the yields are shown in polymerization, but enzyme which was not consumed in the polymerization was again subjected to subsequent cross-linking polymerization so that the enzyme provides the water-soluble cross-linked polymer of the present invention wastelessly without being damaged under mild conditions for polymerization.

Reference example 1

A liquid medium (pH 7.2) containing 1% of pepton, 0.5% of milk casein, 1.0% of saccharose, 0.01% of $K_2HPO_4$ and 0.01% of $MgSO_4 \cdot 7H_2O$ was separately charged by 100 ml each in a 500 ml volume Sakaguchi flask. After sterilization, *Achromobacter lyticus* M497-1 was inoculated and cultivation was performed at 28°C for 24 hours to prepare a seed culture solution. This seed culture solution, 1.5 l, was transplanted to a fermentation tank having charged 30 l of the same medium composition and aeration spinner culture was performed for 4 days while feeding 15 l/min of air at 28°C. After cooling 30 l of the obtained culture solution to about 15°C, bacteria were removed using a centrifuge to obtain about 26 l of the supernatant. To the supernatant, 260 ml of a 4% benzalkonium chloride solution was gradually added dropwise while mildly stirring. After allowing to stand at 4°C for 1 hour, the formed precipitate was removed using a centrifuge to obtain about 25.5 l of the supernatant. To the thus-obtained supernatant (4°C), 80 l of acetone cooled to −5°C was gradually added while mildly stirring. After further allowing to stand in the cold overnight, the formed precipitate was collected using a centrifuge and washed with cold acetone to obtain about 40 g of wet precipitate. After drying this wet precipitate with air, the precipitate was dried for 2 days under reduced pressure in a desiccator laid with silica gel to obtain about 14 g of gray white powdery crude enzyme specimen. The amidase activity of the grude enzyme specimen to Bz-Lys-p-NA was 21.6 units/g. In a 10 mM tris-hydrochloride buffer solution (pH 8.0) 10 g of this acetone powder was dissolved and to 500 ml of the resulting crude enzyme solution, 200 g (wet weight) of carboxymethyl cellulose previously equilibrated with a 10 mM tris-hydrochloride buffer solution (pH 8.0) was added. After stirring the mixture mildly for about 1 hour, the mixture was filtered using a glass filter. The ion exchange cellulose on the filter was washed with a suitable amount of the same buffer solution and the washed liquid was combined with the previous filtrate to obtain 725 ml of un enzyme solution. To 725 ml of the obtained enzyme solution, 460 g (wet weight) of diethylaminoethyl cellulose previously equilibrated with the same buffer solution was added. After stirring the mixture at 4°C for 1 hour, filtration and washing were performed in a manner similar described above. The obtained filtrate was condensed using diaflow membrane UM-10. Thereafter, the condensed filtrate was dialyzed thoroughly against a 2 mM tris-hydrochloride buffer solution (pH 8.0) to obtain 492 ml of an enzyme solution. This enzyme solution was added and adsorbed to a column ($\phi 4 \times 21$ cm) of AH-Sepharose®4B (manufactured by Pharmacia Co., Ltd.) equilibrated with a 2 mM tris-hydrochloride buffer solution (pH 8.0). After thoroughly washing with the same buffer solution, elution was carried out with 2 l of the same buffer solution in which the concentration of sodium chloride was linearly increased from 0 to 1 M. The amidase activity portions eluted at about 0.3 M to 0.5 M of the sodium chloride concentration were collected. After thoroughly dialyzing this enzyme solution with a 2 mM tris-hydrochloride buffer solution (pH 8.0), the solution was condensed using diaflow membrane UM-10 to obtain 10 ml of a concentrated liquid. This concentrated liquid contained protease I, in addition to protease Ia. The amidase activity of this liquid was 14.4 units/ml.

Example 1

The enzyme liquid, 10 ml, obtained in Reference Example 1 was charged in a focusing electrophoresis device (inner volume of 150 ml) filled up with Ampholite® (manufactured by LKB Co., Ltd.) having a pH 3.5 to 10 to subject to isoelectric point fractionation at 4°C for 48 hours at 600 v. After completion of the electrophoresis, fractionation was carried out by 1.6 ml each to measure the amidase activity of the respective fractions. The results as shown in Figure 1 were obtained. As is clear from Figure 5, two peaks of the amidase activity are present. Of these, the latter larger amidase activity peak corresponds to protease I. It is understood that among the peaks having the maximum amdiase activity at the about 46th of the elution fractions, i.e., two activity peaks, the former peak is not negligible. The desired enzyme is contained in this peak fraction and 12.6 ml of this fraction was pooled. The amidase activity was 18.9 μ, specific activity (μ/OD280) was 12.9 and the yield was 8.7% (from acetone powder).

Example 2

After dialyzing the enzyme solution obtained in Example 1 with a 2 mM tris-hydrochloride solution (pH 8.0), condensation was carried out. In order to remove impure proteins present still in a trace amount, electrophoresis was repeated using Ampholine® (pH 4 to 6) under the same conditions as in Example 1 to obtain the results shown in Figure 2. Fractions corresponding to Fraction Numbers 65 to 76 were collected. In order to remove Ampholite® co-present with this enzyme solution, the enzyme solution was passed through a column (φ2×50 cm) of Sephadex®G-50 equilibrated with a 2 mM tris-hydrochloride buffer solution (pH 8.0). After collecting the amidase activity fractions, the fractions were condensed to obtain 5.4 ml of a purified enzyme solution. The amidase activity was 16.1 units. The specific activity (μ/OD280) was 2.24 and the yield was 7.4% (from acetone powder). The thus-purified protease Ia of the present invention underwent electrophoresis as a single protein by analysis of a disc electrophoresis method.

This bacteria, *Achromobacter lyticus* M497-1 was deposited in Fermentation Research Institute, Agency of Industrial Science and Technology. The deposition number is the accession number 6718 (FERM P-6718).

Example 3

After neutralizing 100 mg (2 mM) of porcine insulin and 3.08 g (2 M) of L-threonine-tertiary butyl ester (Thr-OBu$^t$) with 3.5 ml of 5 M acetic acid, 1 M acetate buffer solution is added thereto to dissolve and the total volume was made 8.5 ml (pH 7.0). Thereto, 0.3 ml of a 1 M acetate buffer solution (pH 7.0) containing 1 mg of Achromobacter protease Ia was added and the mixture was kept at 37°C overnight. It is confirmed by high speed liquid chromatography that the desired compound, insulin having threonine protected with tertiary butyl at $B_{30}$ thereof [(Thr-OBu$^t$-$B_{30}$)-insulin], was formed in the yield of 65%.

After rendering the reaction liquid mixture acidic with glacial acetic acid, gel filtration was carried out using a column (40×200 cm) of ultra-finely divided Sephadex®G-50 to fractionate an enzyme fraction, an insulin fraction and a Thr-OBu$^t$ fraction. The enzyme fraction can be reused after dialysis thereof and the Thr-OBu$^t$ fraction after condensation thereof.

After freeze drying, the insulin fraction was passed through a column (2×25 cm) of DEAE Sephadex®A25 equilibrated with 7 M urea. After flowing the aforesaid buffer solution down at 4°C in an amount of 800 ml, elution was carried out with a linear concentration slope up to 3 M of the concentration of sodium chloride to obtain successively fraction A having a concentration of about 0.08 to 0. M and fraction B having a concentration of about 0.13 to 0.14 M. The respective fractions were immediately dialyzed to a 0.01 M ammonium acetate solution in the cold for 3 to 4 days. Thereafter, freeze drying was performed to obtain 55 mg of powders from the fraction A and 35 mg of powders from the fraction B. It is confirmed by high speed liquid chromatography and polyacrylamide gel electrophoresis that the former was [Thr-OBu$^t$-$B_{30}$]-insulin (yield 55%) and the latter was the starting material.

To 50 mg of the obtained [Thr-OBu$^t$-$B_{30}$]-insulin, 2 ml of trifluoroacetic acid containing 0.2 ml of anisole was added and the mixture was kept at room temperature for 30 minutes. Trifluoroacetic acid was removed in a nitrogen flow and anisole was extracted with 15 ml of ether in the presence of 2 ml of 1 N acetic acid. The acetic acid portion was freeze dried to obtain 43 mg of human insulin.

The product was identified to be human insulin by slab gel electrophoresis and high speed liquid chromatogram. Further, the analytical data of amino acids obtained by subjecting the product to hydrolysis with an acid (6 M hydrochloric acid was used and the reaction was at 110°C for 24 hours) are as follows and well identical with calculated values of human insulin as shown in Table 6.

TABLE 6

| Amino acid | Analytical data | | Amino acid | Analytical data | |
|---|---|---|---|---|---|
| | Calculated | Found | | Calculated | Found |
| Lys | 1 | 1.00 | His | 2 | 1.94 |
| Arg | 1 | 0.96 | Asp | 3 | 3.12 |
| Thr | 3 | 3.05 | Ser | 3 | 2.95 |
| Glu | 7 | 7.25 | Gly | 4 | 4.23 |
| Ala | 1 | 1.22 | Val | 4 | 3.86 |
| Ile | 2 | 1.89 | Leu | 6 | 6.13 |
| Thr | 4 | 3.76 | Phl | 3 | 3.12 |

In case that R of Thr-OR being tertiary butyl in the aforesaid example was replaced with methyl, isopropyl, phenethyl and p-methylphenethyl, similar course and results were obtained.

Example 4

After neutralizing 250 mg (5 mM) of porcine insulin and 1 g (0.5 M) of Thr-OBu$^t$ with 1.5 ml of 5 N acetic acid, 6 ml of a 2 M acetate buffer solution and dimethylformamide liquid mixture in 6:4 (volume) was added thereto and pH was adjusted to 6.5. Thereto, 1.5 ml of a 0.2 M acetate buffer solution (pH 6.5) containing 1 mg of Achromobacter protease Ia was added and the mixture was kept at 37°C overnight. It can be confirmed by high speed liquid chromatography that [Thr-OBu$^t$-B$_{30}$]-insulin was formed in the yield of 75%. Then, the product was treated and purified in a manner similar to Example 3 to obtain 150 mg of human insulin. The yield was 60%.

In case that R of Thr-OR was replaced with ethyl, 2-(ethylsulfonyl)ethyl and 2,4,6-trimethylbenzyl, in place of the aforesaid example in which tertiary butyl was used, similar course and results were obtained even when the volume ratio of 2 M acetate buffer solution and dimethylformamide was changed to 5:5 and 8:2 and further the organic solvents were changed to alcohol, glycerol and dioxane.

**Claims for the Contracting States: BE, CH, DE, FR, GB, IT, LI, LU, NL, SE**

1. A process for semi-synthesis of human insulin by preparing human insulin derivatve containing carboxyl group-protected threonine at B$_{30}$ from porcine insulin by using water as a reaction solvent which comprises using Achromobacter protease Ia or water-soluble cross-linked Achromobacter protease Ia.

2. The process of Claim 1, wherein the reaction solvent is water, which solvent may contain in addition one or more organic solvents mixable with water in an amount less than or equal to the volume of water.

3. The process as claimed in Claim 1 or 2, wherein when carboxyl group-protected threonine is represented by Thr-OR, R is substituted or unsubstituted alkyl or aralkyl.

4. The process as claimed in Claims 1 to 3, wherein the reaction is carried out at 50°C or lower.

5. The process as claimed in any one of Claims 1 to 4, wherein the reaction is carried out at a pH of 4 to 10.

6. A process for semi-synthesizing human insulin which comprises preparing a human insulin derivative having carboxyl group-protected threonine at B$_{30}$ by cutting B$_{30}$ alanine from porcine insulin and then reacting the resulting desalanine insulin with threonine having a protected carboxyl group using a water-soluble cross-linked Achromobacter protease Ia.

7. The process for semi-synthesizing human insulin as claimed in Claim 6, wherein, when said carboxyl group-protected threonine is represented by Thr-OR, R is substituted or unsubstituted alkyl or aralkyl.

8. A process for preparing desalanine insulin which comprises cutting B$_{30}$ alanine from porcine insulin using a water-soluble cross-linked Achromobacter protease Ia as a catalyst.

9. Achromobacter protease Ia having the characteristics described below:
(i) molecular weight: 30,000 (determined by a gel filtration method using Sephadex®G-75),
(ii) isoelectric point: 5.3,
(iii) pH reactivity: in esterase activity, optimum pH for the activity is at pH 8.5, and in amidase activity, optimum pH for the activity is at pH 9.0, respectively,
(iv) substrate reactivity: it selectively and specifically hydrolyzes the ester bond and the amide bond at the carboxyl group of L-lysine,
(v) inhibitor: it is inhibited by diisopropyl phosphofluoride, tosyl-L-lysine chloromethyl ketone and phenylmethylsulfonyl chloride,

12

(vi) obtainable from the genus Achromobacter by the following steps which comprise cultivating a bacteria in a culture medium at 20 to 35°C for 1 to 2 days under an aerobic condition, recovering the culture and purifying the enzyme from the culture.

10. A process for preparing a water-soluble cross-linked Achromobacter protease Ia which comprises polymerizing the corresponding Achromobacter protease with a water-soluble protein together with a cross-linking agent, and further with or without the use of at least one spacer.

11. The process as claimed in Claim 10, wherein said cross-linking agent is a polyfunctional organic compound.

12. The process as claimed in Claims 10 and 11, wherein said spacer is polyamine.

13. The process as claimed in Claim 10, 11 and 12, wherein the molecular weight of the modified enzyme is between about 400,000 and about 700,000.

## Claims for the Contracting State: AT

1. A process for semi-synthesis of human insulin by preparing human insulin derivative containing carboxyl group-protected thereonine at $B_{30}$ from porcine insulin by using water as a reaction solvent which comprises using Achromobacter protease Ia or water-soluble cross-linked Achromobacter protease Ia.

2. The process of Claim 1, wherein the reaction solvent is water, which solvent may contain in addition one or more organic solvents mixable with water in an amount less than or equal to the volume of water.

3. The process as claimed in Claim 1 or 2, wherein when carboxyl group-protected threonine is represented by Thr-OR, R is substituted or unsubstituted alkyl or aralkyl.

4. The process as claimed in Claims 1 to 3, wherein the reaction is carried out at 50°C or lower.

5. The process as claimed in any one of Claims 1 to 4, wherein the reaction is carried out at a pH of 4 to 10.

6. A process for semi-synthesizing human insulin which comprises preparing a human insulin derivative having carboxyl group-protected threonine at $B_{30}$ by cutting $B_{30}$ alanine from porcine insulin and then reacting the resulting desalanine insulin with threonine having a protected carboxyl group using a water-soluble cross-linked Achromobacter protease Ia.

7. The process for semi-synthesizing human insulin as claimed in Claim 6, wherein, when said carboxyl group-protected threonine is represented by Thr-OR, R is substituted or unsubstituted alkyl or aralkyl.

8. A process for preparing desalanine insulin which comprises cutting $B_{30}$ alanine from porcine insulin using a water-soluble cross-linked Achromobacter protease Ia as a catalyst.

9. A process for the preparation of Achromobacter protease Ia, characterized in that a protease with the following characteristics:

(i) molecular weight: 30,000 (determined by a gel filtration method using Sephadex®G-75),

(ii) isoelectric point: 5.3,

(iii) pH reactivity: in esterase activity, optimum pH for the activity is at pH 8.5, and in amidase activity, optimum pH for the activity is at pH 9.0, respectively,

(iv) substrate reactivity: it selectively and specifically hydrolyzes the ester bond and the amide bond at the carboxyl group of L-lysine,

(v) inhibitor: it is inhibited by diisopropyl phosphofluoride, tosyl-L-lysine chloromethyl ketone and phenylmethylsulfonyl chloride, is isolated from the genus Achromobacter by the following steps which comprise cultivating a bacteria in a culture medium at 20 to 35°C for 1 to 2 days under an aerobic condition, recovering the culture and purifying the enzyme from the culture.

10. A process for preparing a water-soluble cross-linked Achromobacter protease Ia which comprises polymerizing the corresponding Achromobacter protease with a water-soluble protein together with a cross-linking agent, and further with or without the use of at least one spacer.

11. The process as claimed in Claim 10, wherein said cross-linking agent is a polyfunctional organic compound.

12. The process as claimed in Claims 10 and 11, wherein said spacer is polyamine.

13. The process as claimed in Claims 10, 11 and 12, wherein the molecular weight of the modified enzyme is between about 400,000 and about 700,000.

## Patentansprüche für die Vertragsstaaten BE, CH, DE, FR, GB, IT, LI, LU, NL, SE

1. Ein Verfahren zur Semisynthese von Humaninsulin durch Herstellen eines Humaninsulinderivats, das Carboxylgruppen geschütztes Threonin in der $B_{30}$ Position enthält, aus Schweineinsulin, wobei Wasser als ein Reaktionslösungsmittel verwendet wird, das die Verwendung von Achromobacterprotease Ia oder wasserlöslicher quervernetzter Achromobacterprotease Ia enthält.

2. Verfahren gemäß Anspruch 1, wobei das Reaktionslösungsmittel Wasser ist, dieses Lösungsmittel kann zusätzlich ein oder mehrere organische Lösungsmittel enthalten, die mit Wasser in einer Menge, die geringer oder gleich dem Volumen des Wassers ist, mischbar sind.

3. Verfahren gemäß Anspruch 1 oder 2, wobei, wenn das Carboxylgruppen geschützte Threonin durch Thr-OR dargestellt ist, R substituiertes oder unsubstituiertes Alkyl or Aralkyl ist.

4. Verfahren gemäß Ansprüchen 1 bis 3, wobei die Reaktion bei 50°C oder niedriger ausgeführt wird.

5. Verfahren gemäß irgendeinem der Ansprüche 1 bis 4, wobei die Reaktion bei einem pH von 4 bis 10 durchgeführt wird.

6. Ein Verfahren zur Semisynthese von Humaninsulin, das die Herstellung eines Humaninsulinderivats, das Carboxylgruppen geschütztes Threonin in der $B_{30}$ Poistion hat, enthält, wobei $B_{30}$ Alanin aus Schweineinsulin abgespalten wird und anschließend das sich ergebende Desalanininsulin mit Threonin, das eine geschützte Carboxylgruppe hat, zur Reaktion gebracht wird, wobei eine wasserlösliche quervernetzte Achromobacterprotease Ia verwendet wird.

7. Verfahren zur Semisynthese von Humaninsulin gemäß Anspruch 6, wobei, wenn das Carboxylgruppen geschützte Threonin durch Thr-OR dargestellt ist, R substituiertes oder unsubstitutiertes Alkyl oder Aralkyl ist.

8. Ein Verfahren zur Herstellung von Desalanininsulin, das die Abspaltung des $B_{30}$ Alanins aus Schweineinsulin enthält, wobei eine wasserlösliche quervernetzte Achromobacterprotease Ia als ein Katalysator verwendet wird.

9. Achromobacterproease Ia, die die unten beschriebenen Merkmale hat:

(i) Molekulargewicht: 30.000 (bestimmt mittels einer Gelfiltrationsmethode unter Verwendung von Sephadex®G-75);

(ii) isoelektrischer Punkt: 5.3;

(iii) pH-Reaktivität: im Hindblick auf die Esterase-Aktivität beträgt der optixale pH-Wert für die Aktivität 8,5, und im Hinblick auf die Amidase-Aktivität beträgt der optimale pH-Wert für die Aktivität 9,0;

(iv) Substratreaktivität: sie hydrolysiert selektiv und spezifisch die Esterbindung und die Amidbindung an der Carboxylgruppe des L-Lysins;

(v) Inhibitor: sie wird von Diisopropylphosphofluorid, Tosyl-L-Lysin-chlormethylketon und Phenylmethylsulfonyl-chlorid inhibiert;

(vi) erhältlich aus der Gattung Achromobacter durch die folgenden Schritte, die die Kultivierung einer Bakterie in einem Kulturmedium bei 20 bis 35°C 1 bis 2 Tage unter aerober Bedingung, die Weidergewinnung der Kultur und die Reinigung des Enzyms aus der Kultur enthalten.

10. Verfahren zur Herstellung einer wasserlöslichen quervernetzten Achromobacterprotease Ia, das die Polymerisation der entsprechenden Achromobacterprotease mit einem wasserlöslichen Protein zusammen mit einem quervernetzenden Agens und weiterhin mit oder ohne Verwendung wenigstens eines Spacers enthält.

11. Verfahren gemäß Anspruch 10, wobei das quervernetzende Agens eine polyfunktionelle organische Verbindung ist.

12. Verfahren gemäß Ansprüche 10 und 11, wobei der Spacer Polyamin ist.

13. Verfahren gemäß Ansprüchen 10, 11 und 12, wobei das Molekulargewicht des modifizierten Enzyms zwischen ungefähr 400.000 und ungefähr 700.00 liegt.

**Patentansprüche Für den Vertragsstaat AT**

1. Ein Verfahren zur Semisynthese von Humaninsulin durch Herstellen eines Humaninsulinderivats, das Carboxylgruppen geschütztes Threonin in der $B_{30}$ Position enthält, aus Schweineinsulin, wobei Wasser als ein Reaktionslösungsmittel verwendet wird, das die Verwendung von Achromobacterprotease Ia oder wasserlöslicher quervernetzter Achromobacterprotease Ia enthält.

2. Verfahren gemäß Anspruch 1, wobei das Reaktionslösungsmittel Wasser ist, dieses Lösungsmittel kann zusätzlich ein oder mehrere organische Lösungsmittel enthalten, die mit Wasser in einer Menge, die geringer oder gleich dem Volumen des Wassers ist, mischbar sind.

3. Verfahren gemäß Anspruch 1 oder 2, wobei, wenn das Carboxylgruppen geschützte Threonin durch Thr-OR dargestellt ist, R substituiertes oder unsubstitutiertes Alkyl or Aralkyl ist.

4. Verfahren gemäß Ansprüchen 1 bis 3, wobei die Reaktion bei 50°C oder niedriger ausgeführt wird.

5. Verfahren gemäß irgendeinem der Ansprüche 1 bis 4, wobei die Reaktion bei einem pH von 4 bis 10 durchgeführt wird.

6. Ein Verfahren zur Semisynthese von Humaninsulin, das die Herstellung eines Humaninsulinderivats, das Carboxylgruppen geschütztes Threonin in der $B_{30}$ Position hat, enthält, wobei $B_{30}$ Alanin aus Schweineinsulin abgespalten wird und anschließend das sich ergebende Desalanininsulin mit Threonin, das eine geschützte Carboxylgruppe hat, zur Reaktion gebracht wird, wobei eine wasserlösliche quervernetzte Achromobacterprotease Ia verwendet wird.

7. Verfahren zur Semisynthese von Humaninsulin gemäß Anspruch 6, wobei, wenn das Carboxylgruppen geschützte Threonin durch Thr-OR dargestellt ist, R substituiertes oder unsubstitutiertes Alkyl oder Aralkyl ist.

8. Ein Verfahren zur Herstellung von Desalanininsulin, dsa die Abspaltung des $B_{30}$ Alanins aus Schweineinsulin enthält, wobei eine wasserlösliche quervernetzte Achromobacterprotease Ia als ein Katalysator verwendet wird.

9. Ein Verfahren zur Herstellung von Achromobacterprotease Ia, dadurch gekennzeichnet, daß eine Protease mit den folgenden Merkmalen:

(i) Molekulargewicht: 30.000 (bestimmt mittels einer Gelfiltrationsmethode unter Verwendung von Sephadex®G-75);

(ii) isoelektrischer Punkt: 5.3;

(iii) pH-Reaktivität: in bezug auf die Esterase-Aktivität liegt der optimale pH-Wert für die Aktivität bei pH 8,5, und in bezug auf die Amidase-Aktivität liegt der optimale pH-Wert für die Aktivität bei 9,0;

(iv) Substratreaktivität: sie hydrolysiert selektiv und spezifisch die Esterbindung und die Amidbindung an der Carboxylgruppe des L-Lysins;

(v) Inhibitor: sie wird von Diisopropylphosphofluorid, Tosyl-L-Lysin-chlormethylketon und Phenylmethylsulfonyl-chlorid inhibiert;

aus der Gattung Achromobacter durch die folgenden Schritte isoliert wird, die die Kultivierung einer Bakterie in einem Kulturmedium bei 20 bis 35°C 1 bis 2 Tage unter aerober Bedingung, die Wiedergewinnung der Kultur und die Reinigung des Enzyms aus der Kultur enthalten.

10. Verfahren zur Herstellung einer wasserlöslichen quervernetzten Achromobacterprotease Ia, das die Polymerisation der entsprechenden Achromobacterprotease mit einem wasserlöslichen Protein zusammen mit einem quervernetzenden Agens und weiterhin mit oder ohne Verwendung wenigstens eines Spacers enthält.

11. Verfahren gemäß Anspruch 10, wobei das quervernetzende Agens eine polyfunktionelle organische Verbindung ist.

12. Verfahren gemäß Ansprüchen 10 und 11, wobei der Spacer Polyamin ist.

13. Verfahren gemäß Ansprüchen 10, 11 und 12, wobei das Molekulargewicht des modifizierten Enzyms zwischen ungefähr 400.000 und ungefähr 700.000 liegt.

## Revendications pour les Etats Contractants BE, CH, DE, FR, GB, IT, LI, LU, NL, SE

1. Procédé de semi-synthèse d'insuline humaine, par préparation d'un dérivé d'insuline humaine contenant de la thréonine, à groupe carboxyle protégé en $B_{30}$, à partir d'insuline de porc, en utilisant l'eau comme solvant pour la réaction, ce procédé comprenant l'utilisation d'une protéase Ia d'Achromobacter ou d'une protéase Ia d'Achromobacter, hydrosoluble et réticulée.

2. Procédé selon la revendication 1, dans lequel le solvant utilisé pour la réaction est de l'eau, ce solvant pouvant contenir en outre un ou plusieurs solvants organiques miscibles à l'eau, présents en une quantité inférieure ou égale au volume de l'eau.

3. Procédé tel que revendiqué à la revendication 1 ou 2, dans lequel, lorsque la thréonine à groupe carboxyle protégé est représentée par Thr-OR, R représente un groupe alkyle ou aralkyle substitué ou non substitué.

4. Procédé tel que revendiqué dans les revendications 1 à 3, dans lequel on effectue la réaction à 50°C ou à une température inférieure.

5. Procédé tel que revendiqué dans l'une quelconque des revendications 1 à 4, dans lequel on effectue la réaction à un pH de 4 à 10.

6. Procédé pour effectuer une semi-synthèse d'insuline humaine, ce procédé comprenant la préparation d'un dérivé d'insuline de type humain (insuline humaine), ayant en $B_{30}$ un groupe thréonine à groupe carboxyle protégé, par scission de l'alanine en $B_{30}$ d'une insuline de porc puis réaction de la des-alanine-insuline résultante (insuline sans alanine) avec de la thréonine dont un groupe carboxyle est protégé, le procédé étant réalisé à l'aide d'une protéase Ia d'Achromobacter, qui est hydrosoluble et réticulée.

7. Procédé pour effectuer une semi-synthèse d'insuline humaine, tel que revendiqué à la revendication 6, dans lequel, lorsque ladite thréonine à groupe carboxyle protégé est représentée par Thr-OR, R représente un groupe alkyle ou aralkyle, substitué ou non substitué.

8. Procédé pour préparer de la des-alanine-insuline, qui comprend la scission de l'alanine en $B_{30}$ que l'on enlève d'une insuline porcine, à l'aide d'une protéase Ia d'Achromobacter, hydrosoluble et réticulée, servant de catalyseur.

9. Protéase Ia d'Achromobacter ayant les caractéristiques décrites ci-après:

(i) poids moléculaire: 30 000 (déterminé par une méthode de filtration sur gel, en utilisant "Sephadex"®G-75),

(ii) point isoélectrique: 5,3;

(iii) réactivité en fonction du pH: pour l'activité d'estérase, le pH optimal pour l'activité se situe à pH 8,5 et, pour l'activité d'amidase, le pH optimal pour l'activité se situe à pH 9,0, respectivement,

(iv) réactivé avec un substrat: la protéase hydrolyse sélectivement et spécifiquement la liaison ester et la liaison amide au niveau du groupe carboxyle de la L-lysine;

(v) inhibiteur: la protéase est inhibée par le phosphofluorure de diisopropyle, la tosyl-L-lysine chlorométhylcétone et le chlorure de phénylméthylsulfonyle,

(vi) la protéase peut être obtenue à partir du genre Achromobacter par les étapes suivantes, qui comprennent la culture d'une bactérie dans un milieu de culture à 20 jusqu'à 35°C durant 1 à 2 jours dans un état de culture aérobie, la récupération de la culture et la purification de l'enzyme à partir de la culture.

10. Procédé pour préparer une protéase Ia d'Achromobacter, réticulée et hydrosoluble, qui comprend la polymérisation de la protéase d'Achromobacter correspondante, avec une protéine hydrosoluble et avec un agent de réticulation, et avec ou sans utilisation d'un chaînon ou élément d'espacement.

11. Procédé tel que revendiqué à la revendication 10, dans lequel ledit agent de réticulation est un composé organique polyfonctionnel.

12. Procédé tel que revendiqué aux revendications 10 et 11, dans lequel ledit chaînon d'espacement est une polyamine.

13. Procédé tel que revendiqué aux revendications 10, 11 et 12, dans lequel le poids moléculaire de l'enzyme modifiée se situe entre environ 400 000 et environ 700 000.

**Revendicationspour l'Etat Contractant: AT**

1. Procédé pour effectuer la semi-synthèse d'une insuline de type humain (insuline humaine) par préparation d'un dérivé de l'insuline humaine contenant en $B_{30}$ de la thréonine à groupe carboxyle protégé, que l'on obtient à partir d'une insuline de porc en utilisant de l'eau comme solvant de réaction, ce procédé comprenant l'utilisation d'une protéase la d'Achromobacter ou d'une protéase la Achromobacter, hydrosoluble et réticulée.

2. Procédé selon la revendication 1, dans lequel le solvant pour la réaction est de l'eau et ce solvant peut contenir en outre un ou plusieurs solvants organiques miscibles à l'eau, présent(s) en une quantité inférieure ou égale ou volume de l'eau.

3. Procédé tel que revendiqué à la revendication 1 ou 2, dans lequel, lorsque la thréonine à groupe carboxyle protégé est représentée par Thr-OR, le symbole R représente un groupe alkyle ou aralkyle, substitué ou non substitué.

4. Procédé tel que revendiqué dans les revendications 1 à 3, dans lequel on effectue la réaction à une température égale ou inférieure à 50°C.

5. Procédé tel que revendiqué dans l'une quelconque des revendications 1 à 4, dans lequel on effectue la réaction à un pH de 4 à 10.

6. Procédé pour effectuer la semi-synthèse d'une insuline de type humain (insuline humaine), qui comprend la préparation d'un dérivé d'insuline humaine comportant en $B_{30}$ un fragment thréonine à groupe carboxyle protégé, par scission de l'alanine en $B_{30}$ de l'insuline de porc et en faisant ensuite réagir la des-alanine insuline résultante avec une thréonine ayant un groupe carboxyle protégé, en opérant à l'aide d'une protéase la d'Achromobacter, hydrosoluble et réticulée.

7. Procédé pour effectuer une semi-synthèse d'insuline humaine, tel que revendiqué à la revendication 6, dans lequel, lorsque la thréonine à groupe carboxyle protégé est représentée par Thr-OR, le symbole R représente un groupe alkyle ou aralkyle substitué ou non substitué.

8. Procédé pour préparer de la des-alanine-insuline, qui comprend la scission en $B_{30}$ de l'alanine d'insuline de porc, scission obtenue à l'aide d'une protéase la d'Achromobacter, hydrosoluble et réticulée, servant de catalyser.

9. Procédé pour la préparation d'une protéase la d'Achromobacter, caractérisé en ce qu'on isole une protéase, ayant les caractéristiques suivantes:

(i) poids moléculaire: 30 000 (déterminé par une méthode de filtration sur gel, en utilisant "Sephadex"®G-75),

(ii) point isoélectrique: 5,3;

(iii) réactivé en fonction du pH: pour l'activité d'estérase, le pH optimal pour l'activité se situe à pH 8,5, et, pour l'activité d'amidase, le pH optimal pour l'activité se situe à pH 9,0, respectivement;

(iv) réactivé avec un substrat: la protéase hydrolyse sélectivement et spécifiquement la liaison est et la liaison amide au niveau du groupe carboxyle de la L-lysine;

(v) inhibiteur: la protéase est inhibée par le phosphofluorure de diisopropyle, la tosyl-L-lysine chlorométhylcétone et le chlorure de phénylméthylsulfonyle,

à partir du genre Achromobacter par les étapes suivantes comprenant la culture d'une bactérie dans un milieu de culture entre 20 et 35°C durant 1 à 2 hours dans les conditions d'une culture aérobie, la récupération de la culture et la purification de l'enzyme à partir de la culture.

10. Procédé pour préparer une protéase la d'Achromobacter, hydrosoluble et réticulée, qui comprend la polymérisation de la protéase d'Achromobacter correspondante avec une protéine hydrosoluble et avec un agent de réticulation avec ou sans utiliser un chaînon d'espacement.

11. Procédé tel que revendique à la revendication 10, dans lequel ledit agent de réticulation est un composé organique polyfonctionnel.

12. Procédé tel que revendiqué aux revendications 10 et 11, dans lequel ledit chaînon d'espacement est une polyamine.

13. Procédé tel que revendiqué aux revendications 10, 11 et 12, dans lequel le poids moléculaire de l'enzyme modifiée se situe entre environ 400 000 et environ 700 000.

## FIG. 1

FIG. 2